(19) Europäisches Patentamt
European Patent Office
Office européen des brevets

(11) EP 0 942 938 B1

(12) **EUROPÄISCHE PATENTSCHRIFT**

(45) Veröffentlichungstag und Bekanntmachung des Hinweises auf die Patenterteilung:
**10.09.2003 Patentblatt 2003/37**

(21) Anmeldenummer: **98912440.9**

(22) Anmeldetag: **05.03.1998**

(51) Int Cl.[7]: **C08F 4/60**, C08F 10/00

(86) Internationale Anmeldenummer:
**PCT/EP98/01233**

(87) Internationale Veröffentlichungsnummer:
**WO 98/040416 (17.09.1998 Gazette 1998/37)**

(54) **GETRÄGERTES KATALYSATORSYSTEM, VERFAHREN ZU SEINER HERSTELLUNG UND SEINE VERWENDUNG ZUR POLYMERISATION VON OLEFINEN**

SUPPORTED CATALYST SYSTEM, METHOD FOR THE PRODUCTION AND USE THEREOF IN OLEFIN POLYMERIZATION

SYSTEME DE CATALYSEUR SUR SUPPORT, PROCEDE PERMETTANT DE LE PRODUIRE ET SON UTILISATION POUR LA POLYMERISATION D'OLEFINES

(84) Benannte Vertragsstaaten:
**AT BE DE ES FI FR GB IT NL SE**

(30) Priorität: **07.03.1997 DE 19709402**
**02.04.1997 DE 19713546**
**23.12.1997 DE 19757563**

(43) Veröffentlichungstag der Anmeldung:
**22.09.1999 Patentblatt 1999/38**

(73) Patentinhaber: **Basell Polyolefine GmbH**
**67056 Ludwigshafen (DE)**

(72) Erfinder:
- **BINGEL, Carsten**
  **D-65830 Kriftel (DE)**
- **GOERES, Markus**
  **D-65760 Eschborn (DE)**
- **FRAAIJE, Volker**
  **D-60325 Frankfurt (DE)**
- **WINTER, Andreas**
  **D-61479 Glashütten (DE)**

(56) Entgegenhaltungen:
**EP-A- 0 589 638**
**EP-A- 0 697 419**
**EP-A- 0 704 461**
**WO-A-95/12622**
**WO-A-96/35729**
**WO-A-97/11775**
**US-A- 5 240 894**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 942 938 B1

## Beschreibung

Die vorliegende Erfindung betrifft substituierte Metallocene und hochaktive geträgerte Katalysatorsysteme, die vorteilhaft bei der Olefinpolymerisation eingesetzt werden können und ein Verfahren zu ihrer Herstellung sowie Polymere, die mit den geträgerten Katalysatorsystemen hergestellt werden.

Verfahren zur Herstellung von Polyolefinen mit Hilfe von löslichen, homogenen Katalysatorsystemen, bestehend aus einer Übergangsmetallkomponente vom Typ eines Metallocens und einer Cokatalysatorkomponente vom Typ eines Aluminoxans, einer Lewis-Säure oder einer ionischen Verbindung sind bekannt. Diese. Katalysatoren liefern bei hoher Aktivität Polymere und Copolymere mit enger Molmassenverteilung.

Bei Polymerisationsverfahren mit löslichen, homogenen Katalysatorsystemen bilden sich starke Beläge an Reaktorwänden und am Rührer aus, wenn das Polymer als Feststoff anfällt. Diese Beläge entstehen immer dann durch Agglomeration der Polymerpartikel, wenn Metallocen und/oder Cokatalysator gelöst in der Suspension vorliegen. Die Beläge in den Reaktorsystemen erreichen rasch erhebliche Stärken und besitzen eine hohe Festigkeit. Sie verhindern den Wärmeaustausch zum Kühlmedium und müssen daher regelmäßig entfernt werden. Solche homogenen Katalysatorsysteme sind industriell in flüssigem Monomer oder in der Gasphase nicht einsetzbar.

Zur Vermeidung der Belagbildung im Reaktor sind geträgerte Katalysatorsysteme vorgeschlagen worden, bei denen das Metallocen und/oder die als Cokatalysator dienende Aluminiumverbindung auf einem anorganischen Trägermaterial fixiert werden.

Aus EP-A-0 576 970 sind Metallocene und entsprechende geträgerte Katalysatorsysteme bekannt. Weiterhin offenbaren auch EP-A 697 419 und EP-A 704 461 Katalysatorsysteme, die ein Metallocen, einen Cokatalysator und einen Träger enthalten.

Die geträgerten Katalysatorsysteme liefern bei technisch relevanten Polymerisationtemperaturen von 50 °C bis 80 °C Polymere, insbesondere Polypropylene, mit Schmelzpunkten von maximal 156 °C. Typische Werte für solche Systeme liegen lediglich im Bereich von 150 °C. Für viele Polymeranwendungen, wie bei der Extrusion und Spritzguß, sind solche Produkte bezüglich Härte und mechanischer Festigkeit noch nicht ausreichend.

Es bestand somit die Aufgabe, geträgerte Metallocenkatalysatoren bereitzustellen, die aufgrund ihrer hohen Regio- und Stereospezifität unter technisch relevanten Polymerisationsbedingungen Polymere mit höherem Schmelzpunkt liefern und ein umweltschonendes und wirtschaftliches Verfahren zur Herstellung der Polymere bereitstellen.

Die der vorliegenden Erfindung zugrundeliegende Aufgabe wird durch ein geträgertes Katalysatorsystem gelöst, das mindestens ein speziell substituiertes Metallocen, mindestens einen Cokatalysator, mindestens einen Träger und gegebenenfalls mindestens eine weitere Additivkomponente enthält. Das Katalysatorsystem wird erfindungsgemäß hergestellt, in dem mindestens ein speziell substituiertes Metallocen, mindestens ein Cokatalysator und mindestens ein Träger gemischt werden.

Als Metallocenkomponente des erfindungsgemäßen Katalysatorsystems wird mindestens eine Verbindung der nachstehenden Formel I verwendet

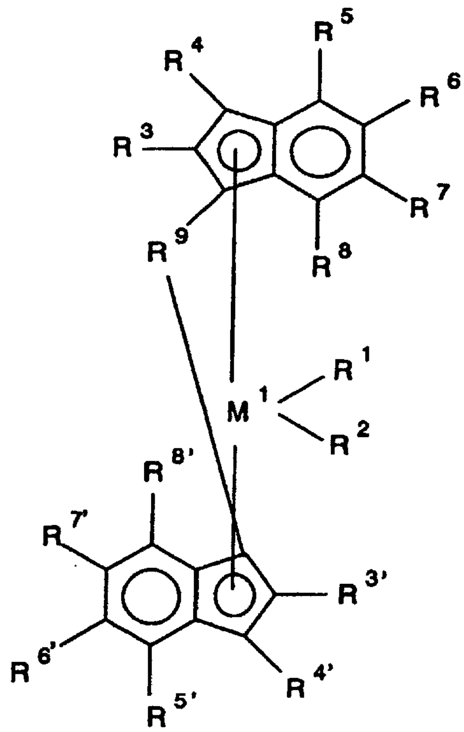

(I)

worin

| | |
|---|---|
| $M^1$ | ein Metall der Gruppe IVb des Periodensystems der Elemente ist, |
| $R^1$ und $R^2$ | gleich oder verschieden sind und ein Wasserstoffatom/ine $C_1$ bis $C_{10}$-Alkylgruppe, eine $C_1$ bis $C_{10}$-Alkoxygruppe, eine $C_6$ bis $C_{20}$-Arylgruppe, eine $C_6$ bis $C_{10}$-Aryloxygruppe, eine $C_2$ bis $C_{10}$-Alkenylgruppe, eine OH-Gruppe, eine $NR^{12}_2$-Gruppe, wobei $R^{12}$ eine $C_1$ bis $C_{10}$-Alkylgruppe oder $C_6$ bis $C_{14}$-Arylgruppe ist, oder ein Halogenatom bedeuten, |
| $R^3$,$R^4$,$R^6$,$R^7$, $R^8$ sowie $R^{3'}$, $R^{4'}$, $R^{6'}$, $R^{7'}$ und $R^{8'}$ | gleich oder verschieden sind und ein Wasserstoffatom, eine Kohlenwasserstoffgruppe, die halogeniert, linear, cyclisch oder verzweigt sein kann, wie eine $C_1$ bis $C_{10}$-Alkylgruppe, $C_2$ bis $C_{10}$-Alkenylgruppe, $C_6$ bis $C_{20}$-Arylgruppe, eine $C_7$ bis $C_{40}$-Arylalkylgruppe, eine $C_7$ bis $C_{40}$-Alkylarylgruppe oder eine $C_8$ bis $C_{40}$-Arylalkenylgruppe bedeuten mit der Maßgabe, daß $R^3$ und $R^{3'}$ von Wasserstoff verschieden sind und |
| $R^5$ und $R^{5'}$ | gleich oder verschieden sind und eine $C_6$ bis $C_{40}$-Arylgruppe der Formel |

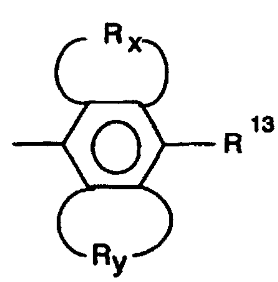

mit x, y = 0, 1 und x + y = 0, 1 oder 2 bedeuten, wobei das aromatische Ringsystem x und/oder das aromatische Ringsystem y auch mit den Resten $R^6$, $R^{6'}$ oder $R^4$, $R^{4'}$ verknüpft sein kann und die $C_6$ bis $C_{40}$-Arylgruppe in para-Position zur Bindungsstelle an den Indenylring einen Substituenten $R^{13}$ trägt und $R^{13}$ tert.-Butyl, Adamantyl oder $(F_3C)_3C$ ist,

$R^9$ eine Verbrückung

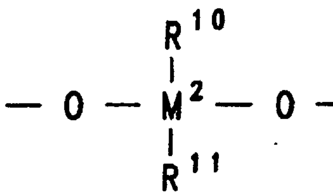, 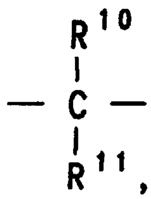, 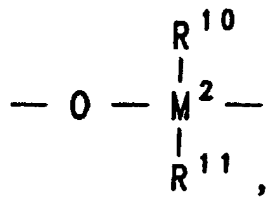, 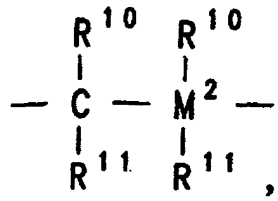,

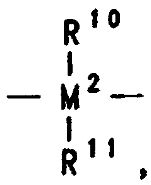, 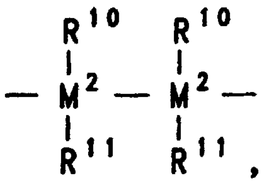, 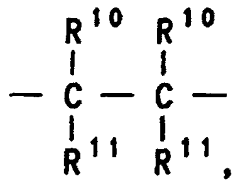, 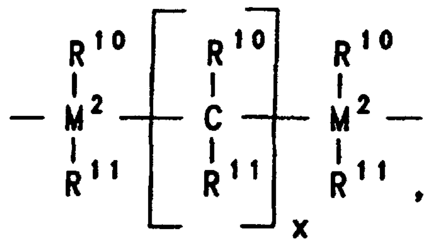,

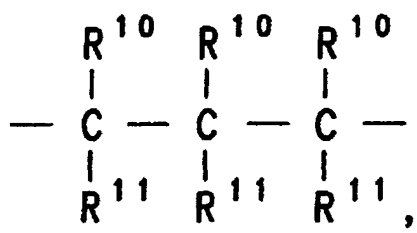,

$>BR^{10}$, $>AlR^{10}$, -Ga-, -O-, -S-, $>SO$, $>SO_2$, $>NR^{10}$, $>OO$, $>PR^{10}$ oder $>R(O)R^{10}$,
bedeutet, wobei

$R^{10}$ und $R^{11}$ auch bei gleicher Indizierung, gleich oder verschieden sein können und ein Wasserstoffatom, ein Halogenatom oder eine $C_1$ bis $C_{40}$-kohlenstoffhaltige Gruppe bedeuten, wie eine $C_1$ bis $C_{20}$-Alkyl , eine $C_1$ bis $C_{10}$-Fluoralkyl-, eine $C_1$ bis $C_{10}$-Alkoxy-, eine $C_6$ bis $C_{14}$-Aryl-, eine $C_6$ bis $C_{10}$-Fluoraryl-, eine $C_6$ bis $C_{10}$-Aryloxy-, eine $C_2$ bis $C_{10}$-Alkenyl-, eine $C_7$ bis $C_{40}$-Arylalkyl-, eine $C_7$ bis $C_{40}$-Alkylaryl- oder eine $C_8$ bis $C_{40}$-Arylalkenylgruppe oder $R^{10}$ und $R^{11}$ bilden jeweils mit den sie verbindenden Atomen einen oder mehrere Ringe, z bedeutet eine ganze Zahl von Null bis 18 ist und

$M^2$ Silizium, Germanium oder Zinn bedeutet und

$R^9$ auch zwei Einheiten der Formel I miteinander verknüpfen kann.

In Formel I gilt bevorzugt, daß

$M^1$ zirkonium, Hafnium oder Titan ist,

$R^1$ und $R^2$ gleich sind und für Methyl oder Chlor stehen,

$R^3$ und $R^{3'}$ gleich oder verschieden sind und eine Kohlenwasserstoffgruppe, die halogeniert, linear, cyclisch oder verzweigt sein kann, wie eine $C_1$ bis $C_{10}$-Alkylgruppe, $C_2$-$C_{10}$-Alkenylgruppe, eine $C_7$-$C_{40}$-Alkylarylgruppe bedeuten,

$R^9$ $R^{10}R^{11}Si=$, $R^{10}R^{11}Ge=$, $R^{10}R^{11}C=$ oder $-(R^{10}R^{11}C-CR^{10}R^{11})$ bedeutet, wobei $R^{10}$ und $R^{11}$ gleich oder verschieden sind und eine $C_1$-$C_{20}$-Kohlenwasserstoffgruppe, insbesondere $C_1$-$C_{10}$-Alkyl oder $C_6$-$C_{14}$-Aryl bedeuten,

$R^5$ und $R^{5'}$ gleich oder verschieden sind und eine $C_6$ bis $C_{20}$-Arylgruppe bedeuten, die in para-Position zur Bindungsstelle an den Indenylring einen Substituenten $R^{13}$ trägt.

In Formel I gilt ganz besonders bevorzugt, daß

| | |
|---|---|
| $M^1$ | Zirkonium ist, |
| $R^1$ und $R^2$ | gleich sind und für Methyl oder Chlor, insbesondere Chlor, stehen, |
| $R^9$ | $R^{10}R^{11}Si=$, $R^{10}R^{11}C=$ oder $-(R^{10}R^{11}C-CR^{10}R^{11})-$ ist, worin $R^{10}$ und $R^{11}$ gleich oder verschieden sind und Wasserstoff, Phenyl, Methyl oder Ethyl bedeuten, |
| $R^4$,$R^6$,$R^7$ und $R^8$ sowie $R^{4'}$, $R^{6'}$,$R^{7'}$und $R^{8'}$ | Wasserstoff sind, und |
| $R^5$ und $R^{5'}$ | gleich oder verschieden sind und eine in para-Position substituierte Phenyl-, Naphthyl- oder Anthracenylgruppe bedeuten. |

Bevorzugte Metallocenkomponenten des erfindungsgemäßen Katalysatorsystems sind Kombinationen folgender Molekülfragmente der Verbindung I

| | |
|---|---|
| $M^1R^1R^2$ | $ZrCl_2$, $Zr(CH_3)_2$, |
| $R^3$,$R^{3'}$ | Methyl, Ethyl, Isopropyl, Isobutyl, n-Butyl, s-Butyl, |
| $R^4$,$R^8$,$R^{4'}$,$R^{8'}$ | Wasserstoff |
| $R^6$,$R^7$,$R^{6'}$,$R^{7'}$ | Wasserstoff, $C_1$ bis $C_4$-Alkyl, $C_6$ bis $C_{10}$-Aryl, |
| $R^5$ und $R^{5'}$ | p-tert.-Butyl-phenyl, p-Adamantyl-phenyl, p-$(F_3C)_3$C-phenyl und |
| $R^9$ | Dimethylsilandiyl, Dimethylgermandiyl, Ethyliden, 1-Methylethyliden, 1,1-Dimethylethyliden, 1,2-Dimethylethyliden, 1,1,2,2-Tetramethylethyliden, Dimethylmethyliden. |

Besonders bevorzugte Metallocenkomponenten des erfindungsgemäßen Katalysatorsystems sind somit folgende Verbindungen I

Dimethylsilandiylbis(2-methyl-4-(p-tert.-butylphenyl)-indenyl)$ZrCl_2$ Dimethylsilandiylbis(2-methyl-4-(p-adamantylphenyl)-indenyl)$ZrCl_2$ Dimethylsilandiylbis(2-methyl-4-(p-tris(trifluormethyl)methylphenyl)-indenyl)$ZrCl_2$ sowie die entsprechenden Dimethylgermandiyl, Ethyliden, 1-Methylethyliden, 1,1-Dimethylethyliden, 1,2-Dimethylethyliden, 1,1,2,2-Tetramethylethyliden und Dimethylmethyliden verbrückten Verbindungen.

Besonders bevorzugte Metallocenkomponenten sind ferner die entsprechenden 2-Ethyl, 2-Isopropyl, 2-Isobutyl, 2-n-Butyl, 2-s-Butyl substituierten Homologen der zuvor genannten Verbindungen I. Herstellungsverfahren für Metallocene der Formel I sind z.B. in Journal of Organometallic Chem. 288 (1985) 63 - 67 und in den dort zitierten Dokumenten beschrieben.

Das erfindungsgemäße Katalysatorsystem enthält mindestens einen Cokatalysator, der mindestens eine Verbindung vom Typ eines Aluminoxans oder einer Lewis-Säure oder einer ionischen Verbindung, die durch Reaktion mit einem Metallocen dieses in eine kationische Verbindung überführt, sein kann.

Als Aluminoxan wird bevorzugt eine Verbindung der allgemeinen Formel II

$$(R\ AlO)_n \qquad (II)$$

verwendet. Aluminoxane können z.B. cyclisch wie in Formel III

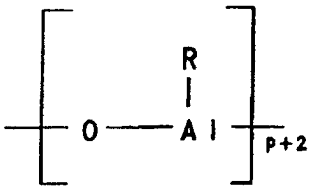

(III)

oder linear wie in Formel IV

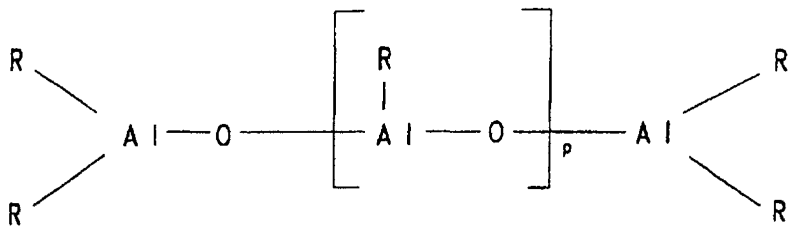

(IV)

oder vom Cluster-Typ wie in Formel V sein, wie sie in neuerer Literatur beschrieben werden, vgl. JACS 117 (1995), 6465-74, Organometallics 13 (1994), 2957-2969.

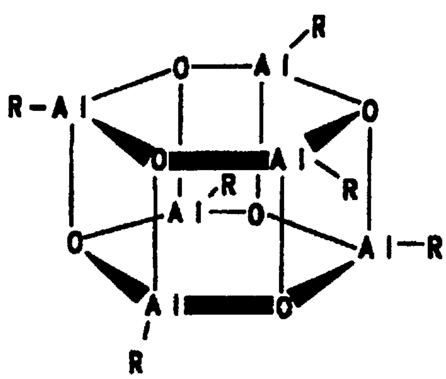

(V)

Die Reste R in den Formeln (II), (III), (IV) und (V) können gleich oder verschieden sein und eine $C_1$ bis $C_{20}$-Kohlenwasserstoffgruppe, wie eine $C_1$ bis $C_6$-Alkylgruppe, eine $C_6$ bis $C_{18}$-Arylgruppe, Benzyl oder Wasserstoff bedeuten und p eine ganze Zahl von 2 bis 50, bevorzugt 10 bis 35 bedeuten.

Bevorzugt sind die Reste R gleich und bedeuten Methyl, Isobutyl, n-Butyl, Phenyl oder Benzyl, besonders bevorzugt Methyl.

Sind die Reste R unterschiedlich, so sind sie bevorzugt Methyl und Wasserstoff, Methyl und Isobutyl oder Methyl und n-Butyl, wobei Wasserstoff, Isobutyl oder n-Butyl bevorzugt zu 0,01 bis 40 % (Zahl der Reste R) enthalten sind.

Das Aluminoxan kann auf verschiedene Arten nach bekannten Verfahren hergestellt werden. Eine der Methoden ist, daß eine Aluminiumkohlenwasserstoffverbindung und/oder eine Hydridoaluminiumkohlenwasserstoffverbindung mit Wasser, gasförmig, fest, flüssig oder als Kristallwasser gebunden in einem inerten Lösungsmittel, wie Toluol umgesetzt wird. Zur Herstellung eines Aluminoxans mit verschiedenen Alkylgruppen R werden entsprechend der gewünschten Zusammensetzung und Reaktivität zwei verschiedene Aluminiumtrialkyle ($AlR_3$ + $AlR'_3$) mit Wasser

umgesetzt, vgl. S. Pasynkiewicz, Polyhedron 9 (1990) 429 und EP-A-0 302 424.

Unabhängig von der Art der Herstellung ist allen Aluminoxanlösungen ein wechselnder Gehalt an nicht umgesetzter Aluminiumausgangsverbindung, die in freier Form oder als Addukt vorliegt, gemeinsam.

Als Lewis-Säure werden bevorzugt mindestens eine bor- oder aluminiumorganische Verbindung eingesetzt, die $C_1$ bis $C_{20}$-kohlenstoffhaltige Gruppen enthalten, wie verzweigte oder unverzweigte Alkyl- oder Halogenalkyl, wie Methyl, Propyl, Isopropyl, Isobutyl, Trifluormethyl, ungesättigte Gruppen, wie Aryl oder Halogenaryl, wie Phenyl, Tolyl, Benzylgruppen, p-Fluorophenyl, 3,5-Difluorophenyl, Pentachlorophenyl, Pentafluorophenyl, 3,4,5 Trifluorophenyl und 3,5 Di(trifluoromethyl)phenyl.

Bevorzugte Lewis Säuren sind Trimethylaluminium, Triethylaluminium, Triisobutylaluminium, Tributylaluminium, Trifluoroboran, Triphenylboran,
Tris(4 fluorophenyl)boran, Tris(3,5 difluorophenyl)boran,
Tris(4 fluoromethylphenyl)boran, Tris(pentafluorophenyl)boran,
Tris(tolyl)boran, Tris(3,5 dimethylphenyl)boran,
Tris(3,5 difluorophenyl)boran und/oder Tris(3,4,5 trifluorophenyl)boran.

Insbesondere bevorzugt ist Tris(pentafluorophenyl)boran.

Als ionische Cokatalysatoren werden bevorzugt Verbindungen eingesetzt, die ein nicht koordinierendes Anion enthalten, wie Tetrakis(pentafluorophenyl)borate, Tetraphenylborate, $SbF_6^-$, $CF_3SO_3^-$ oder $ClO_4^-$. Als kationisches Gegenion werden Lewis-Basen, wie Metyhiamin, Anilin, Dimethylamin, Diethylamin, N-Methylanilin, Diphenylamin, N, N-Dimethylanilin, Trimethylamin, Triethylamin, Tri-n-butylamin, Methyldiphenylamin, Pyridin, p-Bromo-N,N-dimethylanilin, p-Nitro-N,N-dimethylanilin, Triethylphosphin, Triphenylphosphin, Diphenylphosphin, Tetrahydrothiophen und Triphenylcarbenium eingesetzt.

Erfindungsgemäßen ionischen Verbindungen sind Triethylammoniumtetra(phenyl)borat,
Tributylammoniumtetra(phenyl)borat,
Trimethylammoniumtetra(tolyl)borat,
Tributylammoniumtetra(tolyl)borat,
Tributylammoniumtetra(pentafluorophenyl)borat,
Tributylammoniumtetra(pentafluorophenyl)aluminat,
Tripropylammoniumtetra(dimethylphenyl)borat,
Tributylammoniumtetra(trifluoromethylphenyl)borat,
Tributylammoniumtetra(4 fluorophenyl)borat,
N,N Dimethylaniliniumtetra(phenyl)borat,
N,N Diethylaniliniumtetra(phenyl)borat,
N,N Dimethylaniliniumtetrakis(pentafluorophenyl)borate,
N,N Dimethylaniliniumtetrakis(pentafluorophenyl)aluminat,
Di(propyl)ammoniumtetrakis(pentafluorophenyl)borat,
Di(cyclohexyl)ammoniumtetrakis(pentafluorophenyl)borat,
Triphenylphosphoniumtetrakis(phenyl)borat,
Triethylphosphoniumtetrakis(phenyl)borat,
Diphenylphosphoniumtetrakis(phenyl)borat,
Tri(methylphenyl)phosphoniumtetrakis(phenyl)borat,
Tri(dimethylphenyl)phosphoniumtetrakis(phenyl)borat,
Triphenylcarbeniumtetrakis(pentafluorophenyl)borat,
Triphenylcarbeniumtetrakis(pentafluorophenyl)aluminat,
Triphenylcarbeniumtetrakis(phenyl)aluminat,
Ferroceniumtetrakis(pentafluorophenyl)borat und/oder
Ferroceniumtetrakis(pentafluorophenyl)aluminat.

Bevorzugt sind Triphenylcarbeniumtetrakis(pentafluorophenyl)borat und/oder
N,N Dimethylaniliniumtetrakis(pentafluorophenyl)borat.

Es können auch Gemische mindestens einer Lewis Säure und mindestens einer ionischen Verbindung eingesetzt werden.

Als Cokatalysatorkomponenten sind ebenfalls Boran oder Carboran Verbindungen wie 7,8 Dicarbaundecaboran(13), Undecahydrid 7,8 dimethyl 7,8 dicarbaundecaboran,
Dodecahydrid 1 phenyl 1,3 dicarbanonaboran,
Tri(butyl)ammoniumundecahydrid 8 ethyl 7,9 dicarbaundecaborat, 4 Carbanonaboran(14),
Bis(tri(butyl)ammonium)nonaborat,
Bis(tri(butyl)ammonium)undecaborat,
Bis(tri(butyl)ammonium)dodecaborat,
Bis(tri(butyl)ammonium)decachlorodecaborat,

Tri(butyl)ammonium 1 carbadecaborate,
Tri(butyl)ammonium 1 carbadodecaborate,
Tri(butyl)ammonium 1 trimethylsilyl 1 carbadecaborate,
Tri(buyl)ammoniumbis(nonahydrid 1,3 dicarbonnonaborat)cobaltate(III),
Tri(butyl)ammoniumbis(undecahydrid 7,8 dicarbaundecaborat) ferrat(III)
von Bedeutung.

Die Trägerkomponente des erfindungsgemäßen Katalysatorsystems kann ein beliebiger organischer oder anorganischer, inerter Feststoff sein, insbesondere ein poröser Träger, wie Talk, anorganische Oxide und feinteilige Polymerpulver, Polyolefine.

Geeignete anorganische Oxide finden sich in den Gruppen 2, 3, 4, 5, 13, 14, 15 und 16 des Periodensystems der Elemente. Bevorzugte Oxide für Träger sind Siliciumdioxid, Aluminiumoxid sowie Mischoxide der beiden Elemente und entsprechende Oxid-Mischungen. Andere anorganische Oxide, die allein oder in Kombination mit den zuletzt genannten bevorzugten oxidischen Trägern eingesetzt werden können, sind auch MgO, $ZrO_2$, $TiO_2$ oder $B_2O_3$.

Die verwendeten Trägermaterialien weisen eine spezifische Oberfläche im Bereich von 10 bis 1000 $m^2/g$, ein Porenvolumen im Bereich von 0,1 bis 5 ml/g und eine mittlere Partikelgröße von 1 bis 500 µm auf. Bevorzugt sind Träger mit einer spezifischen Oberfläche im Bereich von 50 bis 500 $m^2/g$, einem Porenvolumen im Bereich zwischen 0, 5 und 3, 5 ml/g und einer mittleren Partikelgröße im Bereich von 5 bis 350 µm. Besonders bevorzugt sind Träger mit einer spezifischen Oberfläche im Bereich von 200 bis 400 $m^2/g$, einem Porenvolumen im Bereich zwischen 0,8 bis 3,0 ml/g und einer mittleren Partikelgröße von 10 bis 200 µm.

Wenn das verwendete Trägermaterial von Natur aus einen geringen Feuchtigkeitsgehalt oder Restlösemittelgehalt aufweist, kann eine Dehydratisierung oder Trocknung vor der Verwendung unterbleiben. Ist dies nicht der Fall, wie bei dem Einsatz von Silicagel als Trägermaterial, ist eine Dehydratisierung oder Trocknung empfehlenswert. Die thermische Dehydratisierung oder Trocknung des Trägermaterials kann unter Vakuum und gleichzeitiger Inertgasüberlagerung (Stickstoff) erfolgen. Die Trocknungstemperatur liegt im Bereich zwischen 100 °C und 1000 °C, vorzugsweise zwischen 200 °C und 800 °C. Der Parameter Druck ist in diesem Fall nicht entscheidend. Die Dauer des Trocknungsprozesses kann zwischen 1 und 24 Stunden betragen. Kürzere oder längere Trocknungsdauern sind möglich, vorausgesetzt, daß unter den gewählten Bedingungen die Gleichgewicntseinstellung mit den Hydroxylgruppen auf der Trägeroberfläche erfolgen kann, was normalerweise zwischen 4 und 8 Stunden erfordert.

Eine Dehydratisierung oder Trocknung des Trägermaterials ist auch auf chemischem Wege möglich, in dem das adsorbierte Wasser und die Hydroxylgruppen auf der Oberfläche mit geeigneten Inertisierungsmitteln zur Reaktion gebracht werden. Durch die Umsetzung mit dem Inertisierungsreagenz können die Hydroxylgruppen vollständig oder auch teilweise in eine Form überführt werden, die zu keiner negativen Wechselwirkung mit den katalytisch aktiven Zentren führen. Geeignete Inertisierungsmittel sind Siliciumhalogenide und Silane, wie Siliciumtetrachlorid, Chlortrimethylsilan, Dimethylaminotrichlorsilan oder metallorganische Verbindungen von Aluminium-, Bor und Magnesium, wie Trimethylaluminium, Triethylaluminium, Triisobutylaluminium, Triethylboran, Dibutylmagnesium. Die chemische Dehydratisierung oder Inertisierung des Trägermaterials erfolgt dadurch, daß man unter Luft- und Feuchtigkeitsausschluß eine Suspension des Trägermaterials in einem geeigneten Lösemittel mit dem Inertisierungsreagenz in reiner Form oder gelöst in einem geeigneten Lösemittel zur Reaktion bringt. Geeignete Lösemittel sind aliphatische oder aromatische Kohlenwasserstoffe, wie Pentan, Hexan, Heptan, Toluol oder Xylol. Die Inertisierung erfolgt bei Temperaturen zwischen 25 °C und 120 °C, bevorzugt zwischen 50 °C und 70 °C. Höhere und niedrigere Temperaturen sind möglich. Die Dauer der Reaktion beträgt zwischen 30 Minuten und 20 Stunden, bevorzugt 1 bis 5 Stunden. Nach dem vollständigen Ablauf der chemischen Dehydratisierung wird das Trägermaterial durch Filtration unter Inertbedingungen isoliert, ein- oder mehrmals mit geeigneten inerten Lösemitteln, wie sie bereits zuvor beschrieben worden sind gewaschen und anschließend im Inertgasstrom oder am Vakuum getrocknet.

Organische Trägermaterialien, wie feinteilige Polyolefinpulver, wie Polyethylen, Polypropylen oder Polystyrol können auch verwendet werden und sollten ebenfalls vor dem Einsatz von anhaftender Feuchtigkeit, Lösemittelresten oder anderen Verunreinigungen durch entsprechende Reinigungs- und Trocknungsoperationen befreit werden.

Zur Darstellung des geträgerten Katalysatorsystems wird mindestens eine der oben beschriebenen Metallocenkomponenten in einem geeigneten Lösemittel mit mindestens einer Cokatalysatorkomponente in Kontakt gebracht, wobei bevorzugt ein lösliches Reaktionsprodukt, ein Addukt oder ein Gemisch erhalten wird.

Die so erhaltene Zubereitung wird dann mit dem dehydratisierten oder inertisierten Trägermaterial vermischt, das Lösemittel entfernt und das resultierende geträgerte Metallocenkatalysatorsystem getrocknet, um sicherzustellen, daß das Lösemittel vollständig oder zum größten Teil aus den Poren des Trägermaterials entfernt wird. Der geträgerte Katalysator wird als frei fließendes Pulver erhalten.

Ein Verfahren zur Darstellung eines frei fließenden und gegebenenfalls vorpolymerisierten geträgerten Katalysatorsystems umfaßt die folgenden Schritte

a) Herstellung einer Metallocen/Cokatalysatormischung in einem geeigneten Löse- oder Suspensionsmittel, wobei

die Metallocenkomponente eine der zuvor beschriebenen Strukturen besitzt

b) Aufbringen der Metallocen/Cokatalysatormischung auf einen porösen, bevorzugt anorganischen dehydratisierten Träger,

c) Entfernen des Hauptanteils an Lösemittel von der resultierenden Mischung

d) Isolierung des geträgerten Katalysatorsystems

e) Gegebenenfalls eine Vorpolymerisation des erhaltenen geträgerten Katalysatorsystems mit einem oder mehreren olefinischen Monomer(en), um ein vorpolymerisiertes geträgertes Katalysatorsystem zu erhalten.

Bevorzugte Lösemittel für die Herstellung der Metallocen/Cokatalysatormischung sind Kohlenwasserstoffe und Kohlenwasserstoffgemische, die bei der gewählten Reaktionstemperatur flüssig sind und in denen sich die Einzelkomponenten bevorzugt lösen. Die Löslichkeit der Einzelkomponenten ist aber keine Voraussetzung, wenn sichergestellt ist, daß das Reaktionsprodukt aus Metallocen- und Cokatalysatorkomponenten in dem gewählten Lösemittel löslich ist. Geeignete Lösemittel sind Alkane, wie Pentan, Isopentan, Hexan, Heptan, Octan und Nonan, Cycloalkane, wie Cyclopentan und Cyclohexan und Aromaten, wie Benzol, Toluol, Ethylbenzol und Diethylbenzol. Ganz besonders bevorzugt ist Toluol.

Die bei der Präparation des geträgerten Katalysatorsystems eingesetzten Mengen an Aluminoxan und Metallocen können über einen weiten Bereich variiert werden. Bevorzugt wird ein molares Verhältnis von Aluminium zum Übergangsmetall im Metallocen von 10 : 1 bis 1000 : 1 eingestellt, ganz besonders bevorzugt ein Verhältnis von 50 : 1 bis 500 : 1. Im Fall von Methylaluminoxan werden bevorzugt 30 % ige toluolische Lösungen eingesetzt, die Verwendung von 10 %igen Lösungen ist aber auch möglich.

Zur Voraktivierung wird das Metallocen in Form eines Feststoffes in einer Lösung des Aluminoxans in einem geeigneten Lösemittel aufgelöst. Es ist auch möglich, das Metallocen getrennt in einem geeigneten Lösemittel aufzulösen und diese Lösung anschließend mit der Aluminoxanlösung zu vereinigen. Bevorzugt wird Toluol verwendet. Die Voraktivierungszeit beträgt 1 Minute bis 200 Stunden. Die Voraktivierung kann bei Raumtemperatur von 25 °C stattfinden. Die Anwendung höherer Temperaturen kann im Einzelfall die erforderliche Dauer der Voraktivierung verkürzen und eine zusätzliche Aktivitätssteigerung bewirken. Höhere Temperatur bedeutet in diesem Fall ein Bereich zwischen 50 °C und 100 °C.

Die voraktivierte Lösung oder das Metallocen/Cokatalysatorgemisch wird anschließend mit einem inerten Trägermaterial, üblicherweise Kieselgel, das in Form eines trockenen Pulvers oder als Suspension in einem der oben genannten Lösemittel vorliegt, vereinigt. Bevorzugt wird das Trägermaterial als Pulver eingesetzt. Die Reihenfolge der Zugabe ist dabei beliebig. Die voraktivierte Metallocen/Cokatalysatorlösung oder das Metallocen/Cokatalysatorgemisch kann zum vorgelegten Trägermaterial dosiert, oder aber das Trägermaterial in die vorgelegte Lösung eingetragen werden.

Das Volumen der voraktivierten Lösung oder des Metallocen/Cokatalysatorgemisches kann 100 % des Gesamtporenvolumens des eingesetzten Trägermaterials überschreiten oder aber bis zu 100 % des Gesamtporenvolumens betragen.

Die Temperatur, bei der die voraktivierte Lösung oder das Metallocen/Cokatalysatorgemisch mit dem Trägermaterial in Kontakt gebracht wird, kann im Bereich zwischen 0 °C und 100 °C variieren. Niedrigere oder höhere Temperaturen sind aber auch möglich.

Anschließend wird das Lösemittel vollständig oder zum größten Teil vom geträgerten Katalysatorsystem entfernt, wobei die Mischung gerührt und gegebenenfalls auch erhitzt werden kann. Bevorzugt wird sowohl der sichtbare Anteil des Lösemittels als auch der Anteil in den Poren des Trägermaterials entfernt. Das Entfernen des Lösemittels kann in konventioneller Art und Weise unter Anwendung von Vakuum und/oder Spülen mit Inertgas erfolgen. Beim Trocknungsvorgang kann die Mischung erwärmt werden, bis das freie Lösemittel entfernt worden ist, was üblicherweise 1 bis 3 Stunden bei einer vorzugsweise gewählten Temperatur zwischen 30 °C und 60 °C erfordert. Das freie Lösemittel ist der sichtbare Anteil an Lösemittel in der Mischung. Unter Restlösemittel versteht man den Anteil, der in den Poren eingeschlossen ist.

Alternativ zu einer vollständigen Entfernung des Lösemittels kann das geträgerte Katalysatorsystem auch nur bis zu einem gewissen Restlösemittelgehalt getrocknet werden, wobei das freie Lösemittel vollständig entfernt worden ist. Anschließend kann das geträgerte Katalysatorsystem mit einem niedrig siedenden Kohlenwasserstoff, wie Pentan oder Hexan gewaschen und erneut getrocknet werden.

Das erfindungsgemäß dargestellte geträgerte Katalysatorsystem kann entweder direkt zur Polymerisation von Olefinen eingesetzt oder vor seiner Verwendung in einem Polymerisationsprozeß mit einem oder mehreren olefinischen Monomeren vorpolymerisiert werden. Die Ausführung der Vorpolymerisation von geträgerten Katalysatorsystemen ist in WO 94/28034 beschrieben.

Als Additiv kann während oder nach der Herstellung des geträgerten Katalysacorsystems eine geringe Menge eines Olefins bevorzugt eines α-Olefins, wie Styrol oder Phenyldimethylvinylsilan als aktivitätssteigernde Komponente oder eines Antistatikums, wie in US Serial No. 08/365280 beschrieben, zugesetzt werden. Das molare Verhältnis von Additiv zu Metallocenkomponente Verbindung I beträgt dabei bevorzugt zwischen 1 : 1000 bis 1000 : 1, ganz besonders bevorzugt 1 : 20 bis 20 : 1.

Die vorliegende Erfindung betrifft auch ein Verfahren zur Herstellung eines Polyolefins durch Polymerisation einer oder mehrerer Olefine in Gegenwart des erfindungsgemäßen Katalysatorsystems, enthaltend mindestens eine Übergangsmetallkomponente der Formel I. Unter dem Begriff Polymerisaton wird eine Homopolymerisation, wie auch eine Copolymerisation verstanden.

Bevorzugt werden Olefine der Formel $R_m$-CH=CH-$R_n$ polymerisiert, worin $R_m$ und $R_n$ gleich oder verschieden sind und ein Wasserstoffatom oder einen kohlenstoffhaltigen Rest mit 1 bis 20 C-Atomen, insbesondere 1 bis 10 C-Atome bedeuten und $R_m$ und $R_n$ zusammen mit den sie verbindenden Atomen einen oder mehrere Ringe bilden können.

Geegnete Olefine sind 1-Olefine mit 2 bis 40 vorzugsweise 2 bis 10 C-Atomen, wie Ethen, Propen, 1-Buten, 1-Penten, 1-Hexen, 4-Methyl-1-penten oder 1-Octen, Styrol, Diene, wie 1,3-Butadien, 1,4-Hexadien, Vinylnorborne, Norbornadien, Ethylnorbornadien und cyclische Olefine, wie Norbornen, Tetracyclododecen oder Methylnorbornen. Bevorzugt werden in dem erfindungsgemäßen Verfahren Propen oder Ethen homopolymerisiert oder Propen mit Ethen und/oder mit einem oder mehreren 1-Olefinen mit 4 bis 20 C-Atomen, wie Hexen und/oder einem oder mehreren Dienen mit 4 bis 20 C-Atomen, wie 1,4-Butadien, Norbornadien, Ethylidennorbonen oder Ethylnorbornadien copolymerisiert. Geeignete Copolymere sind Ethen/Propen-Copolymere oder Ethen/Propen/1,4-Hexadien-Terpolymere.

Die Polymerisation wird bei einer Temperatur von - 60 °C bis 300 °C, bevorzugt 50 °C bis 200 °C, ganz besonders bevorzugt 50 °C bis 80 °C durchgeführt. Der Druck beträgt 0,5 bis 2000 bar, bevorzugt 5 bis 64 bar.

Die Polymerisation kann in Lösung, in Masse, in Suspension oder in der Gasphase, kontinuierlich oder diskontinuierlich, ein- oder mehrstufig durchgeführt werden.

Das erfindungsgemäß dargestellte Katalysatorsystem kann als einzige Katalysatorkomponente für die Polymerisation von Olefinen mit 2 bis 20 C-Atomen eingesetzt werden, oder bevorzugt in Kombination mit mindestens einer Alkylverbindung der Elemente aus der I. bis III. Hauptgruppe des Periodensystems, wie einem Aluminium-, Magnesium- oder Lithiumalkyl oder einem Aluminoxan eingesetzt werden. Die Alkylverbindung wird dem Monomeren oder Suspensionsmittel zugesetzt und dient zur Reinigung des Monomeren von Substanzen, die die Katalysatoraktivität beeinträchtigen können. Die Menge der zugesetzten Alkylverbindung hängt von der Qualität der eingesetzten Monomere ab.

Als Molmassenregler und/oder zur Steigerung der Aktivität wird, falls erforderlich, Wasserstoff zugegeben.

Bei der Polymerisation kann außerdem ein Antistatikum zusammen mit oder getrennt von dem eingesetzten Katalysatorsystem in das Polymerisationssystem eindosiert werden.

Die mit dem erfindungsgemäßen Katalysatorsystem dargestellten Polymere zeigen eine gleichmäßige Kornmorphologie und weisen keine Feinkornanteile auf. Bei der Polymerisation mit dem erfindungsgemäßen Katalysatorsystem treten keine Beläge oder Verbackungen auf.

Mit dem erfindungsgemäßen Katalysatorsystem werden Polymere, wie Polypropylen mit außerordentlich hoher Stereo und Regiospezifität erhalten.

Besonders charakteristisch für die Stereo und Regiospezifität von Polymeren, insbesondere von Polypropylen ist die Triaden Taktizität (TT) und der Anteil an 2,1-insertierten Propeneinheiten (RI), die sich aus den $^{13}$C NMR Spektren ermitteln lassen.

Die $^{13}$C NMR Spektren werden in einem Gemisch aus Hexachlorbutadien und Tetrachlorethan $d_2$ bei erhöhter Temperatur, wie 365 K gemessen. Alle $^{13}$C NMR Spektren der gemessenen Polypropylen Proben werden auf das Resonanzsignal von Tetrachlorethan $d_2$ (d = 73.81 ppm) geeicht.

Zur Bestimmung der Triaden Taktizität des Polypropylens werden die Methyl Resonanzsignale im $^{13}$C NMR Spektrum zwischen 23 und 16 ppm betrachtet, vgl. J. C. Randall, Polymer Sequence Determination: Carbon 13 NMR Method, Academic Press New York 1978, A. Zambelli, P. Locatelli, G. Bajo, F. A. Bovey, Macromolucules 8 (1975), 687 689, H. N. Cheng, J. A. Ewen, Makromol. Chem. 190 (1989). 1931 1943. Drei aufeinander folgende 1 2 insertierte Propeneinheiten, deren Methylgruppen in der "Fischer Projektion" auf der gleichen Seite angeordnet sind, bezeichnet man als mm Triade (d =21.0 ppm bis 22.0 ppm). Zeigt nur die zweite Methylgruppe der drei aufeinander folgenden Propeneinheiten zur anderen Seite, spricht man von einer rr Triade (d = 19.5 ppm bis 20.3 ppm) und zeigt nur die dritte Methylgruppe der drei aufeinander folgenden Propeneinheiten zur anderen Seite, von einer mr Triade (d = 20.3 ppm bis 21.0 ppm). Die Triaden Taktizität berechnet man nach folgender Formel

$$TT\ (\%) = mm / (mm + mr + rr) \times 100$$

Wird eine Propeneinheit invers in die wachsende Polymerkette insertiert, spricht man von einer 2,1-Insertion, vgl. T. Tsutsui, N. Ishimaru, A. Mizuno, A. Toyota, N. Kashiwa, Polymer 30, (1989), 1350 56. Folgende verschiedene strukturelle Anordnungen sind möglich:

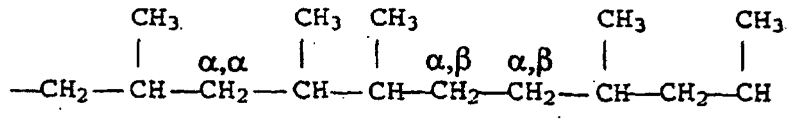

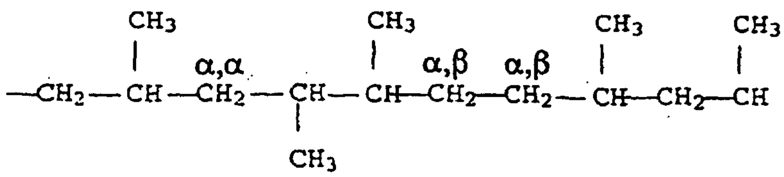

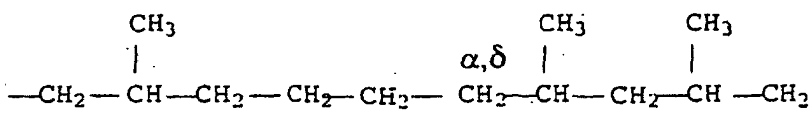

Der Anteil an 2,1-insertierten Propeneinheiten (RI) kann nach folgender Formel berechnet werden

$$RI\ (\%) = 0.5\ Ia,\beta\ (Ia,a + Ia,\beta + Ia,d) \cdot 100,$$

wobei

Ia,a die Summe der Intensitäten der Resonanzsignale bei d = 41.84, 42.92 und 46.22 ppm,
Ia,β die Summe der Intensitäten der Resonanzsignale bei d = 30.13, 32.12, 35.11 und 35.57 ppm

sowie

Ia,d die Intensität des Resonanzsignals bei d = 37.08 ppm bedeuten.

Eine besonders hohe Regiospezifität bedingt auch einen besonders hohen Schmelzpunkt des Polymers, insbesondere des isotaktischen Polypropylens. Das isotaktische Polypropylen, das mit dem erfindungsgemäßen Katalysatorsystem hergestellt worden ist, zeichnet sich durch einen Anteil an 2,1-insertierten Propeneinheiten RI < 0.5% bei einer Triaden Taktizität TT > 98.0% und einen Schmelzpunkt > 156 °C aus, wobei $M_w/M_n$ des erfindungsgemäßen Polypropylens zwischen 2.5 und 3.5 liegt.

Die mit dem erfindungsgemäßen Katalysatorsystem herstellbaren Copolymere zeichnen sich durch eine gegenüber dem Stand der Technik deutlich höhere Molmasse aus. Gleichzeitig sind solche Copolymere durch Einsatz des erfindungsgemäßen Katalysatorsystems mit hoher Produktivität bei technisch relevanten Prozessparametern ohne Belagsbildung herstellbar.

Die nach dem erfindungsgemäßen Verfahren hergestellten Polymere sind insbesondere zur Herstellung reißfester, harter und steifer Formkörper, wie Fasern, Filamente, Spritzgußteile, Folien, Platten oder Großhohlkörpem, wie Rohre geeignet.

Beispiele

Allgemeine Angaben

Die Herstellung und Handhabung der organometallischen Verbindungen erfolgte unter Ausschluß von Luft und Feuchtigkeit unter Argon mit der Schlenk-Technik oder in einer Glove-Box. Alle benötigten Lösemittel wurden vor Gebrauch mit Argon gespült und über Molsieb absolutiert.

Die eingesetzten Metallocene wurden mit $^1$H-NMR, $^{13}$C-NMR und IR-Spektroskopie charakterisiert.

Es bedeuten

| | |
|---|---|
| PP = | Polypropylen |
| MC = | Metallocen |
| Kat = | geträgertes Katalysatorsystem |
| h = | Stunde |
| VZ = | Viskositätszahl in $cm^3/g$ |
| $M_w$ = | Molmassengewichtsmittel in g/mol |
| $M_w/M_n$ = | Molmassenverteilung, ermittelt durch Gelpermeationschromatographie |
| SD = | Schüttdichte in $g/dm^3$ und |
| Smp. = | Schmelzpunkt in °C, ermittelt durch Differential Scanning Calorimetry (DSC) |
| TT = | TriadenTaktizität in Prozent ermittelt durch $^{13}$C-NMR-Spektroskopie |
| RI = | Regiofehler in %, ermittelt durch $^{13}$C-NMR-Spektroskopie |
| $T_g$ = | Glasübergangstemperatur in °C, ermittelt durchDifferentiall Scanning Calorimetry |

Beispiel 1

Darstellung des geträgerten Katalysatorsystems

67 mg (0.091 mmol) rac-Dimethylsilandiylbis(2-methyl-4-(paratert-butyl-phenyl)-indenyl)zirkoniumdichlorid wurden bei Raumtemperatur in 4,3 $cm^3$ (20 mmol Al) 30 %iger toluolischer Methylaluminoxanlösung gelöst. Die Lösung wurde mit 3,7 $cm^3$ Toluol verdünnt und lichtgeschützt bei 25 °C 1 h gerührt. Diese Lösung wurde portionsweise unter Rühren zu 4 g $SiO_2$[2)] gegeben und der Ansatz nach beendeter Zugabe 10 min nachgerührt. Das Verhältnis Volumen der Lösung zum Gesamtporenvolumen des Trägermaterials betrug 1,25. Anschließend wurde der Ansatz innerhalb von 4 h bei 40 °C und $10^{-3}$ mbar getrocknet. Es wurden 5,6 g eines frei fließenden, graubraunen Pulvers erhalten, das laut Elementaranalyse 0.16 Gew% Zr und 9,5 Gew% Al enthielt.

Polymerisation

Ein trockener 16 $dm^3$ -Reaktor, der zunächst mit Stickstoff und anschließend mit Propen gespült worden war, wurde mit 10 $dm^3$ flüssigem Propen gefüllt. Als Scavenger wurden 8 $cm^3$ 20 %iger Triethylaluminiumlösung in Varsol® der Firma Witco zugesetzt und der Ansatz 15 min bei 30 °C gerührt. Anschließend wurde eine Suspension von 2 g des geträgerten Metallocenkatalysators in 20 $cm^3$ Exxsol in den Reaktor gegeben, auf die Polymerisationstemperatur von 65 °C aufgeheizt und das Polymerisationssystem 1 h bei 65 °C gehalten. Die Polymerisation wurde durch Entgasen gestoppt und das erhaltene Polymer im Vakuum getrocknet. Es resultierten 3,2 kg Polypropylenpulver.

Die Katalysatoraktivität betrug 134 kg PP/ (g MC x h) oder 1,6 kg PP /(g Kat x h)

Das dargestellte isotaktische Polypropylen wies die folgenden Eigenschaften auf:

Smp. 159 °C, $M_w$ = 900 000 g/mol, $M_w/M_n$ = 2,6, VZ = 760 $cm^3/g$,
SD = 460 $g/dm^3$, TT = 98,9 %, RI = 0,36 %.

1) Albemarle Corporation, Baton Rouge, Louisiana, USA
2) Silica Typ MS 948 , W.R. Grace, Davison Chemical Devision, Baltimore, Maryland, USA, Porenvolumen 1,6 ml/g, calciniert bei 600 °C

Vergleichsbeispiel 1

Darstellung des geträgerten Katalysatorsystems

Die Darstellung erfolgte analog Beispiel 1, aber mit 58 mg (0.091 mmol) rac-Dimethylsilandiylbis(2-methyl-4-phenylindenyl)zirkoniumdichlorid als Metallocenkomponente. Es wurden 5,8 g eines frei fließenden, rosafarbenen Pulvers erhalten, das laut Elementaranalyse 0.15 Gew% Zr und 9,7 Gew% Al enthielt.

Polymerisation

Die Polymerisation wurde analog zu Beispiel 1 durchgeführt. Es resultierten 2,3 kg Polypropylenpulver.
Die Katalysatoraktivität betrug 115 kg PP / (g MC x h) oder 1,15 kg PP /(g Kat x h).
Das dargestellte isotaktische Polypropylen wies die folgenden Eigenschaften auf:

Smp. 149 °C, $M_w$ = 850 000 g/mol, $M_w/M_n$ = 2,7, VZ = 710 $cm^3$/g,
SD = 420 g/$dm^3$, TT = 98,3 %, RI = 0,8 %.

Beispiel 2

Die Polymerisation erfolgte analog zu Beispiel 1, es wurden jedoch zusätzlich 5 $Ndm^3$ Wassersoff in der Polymerisation eingesetzt und die Polymerisationsdauer war 30 Minuten. Es resultierten 2.9 kg Polypropylenpulver.
Die Katalysatoraktivität betrug 242 kg PP / (g MC x h) oder 2,9 kg PP /(g Kat x h).
Das dargestellte isotaktische Polypropylen wies die folgenden Eigenschaften auf:

Smp. 160 °C, $M_w$ = 450 000 g/mol, $M_w/M_n$ = 3,2, VZ = 370 $cm^3$/g,
SD = 450 g/$dm^3$ TT = 98,9 %, RI = 0,3 %.

Beispiel 3 und 4

Ein trockener 24 $dm^3$ -Reaktor wurde mit Propylen gespült und mit 12 $dm^3$ flüssigem Propylen, 150 g Ethylen (Beispiel 3) beziehungsweise 450 g Ethylen (Beispiel 4) und 22 $cm^3$ einer hexanischen Triisobutylaluminiumlösung (8 mmol Al, 2 $cm^3$ Triisobutylaluminium verdünnt mit 20 $cm^3$ Hexan) befüllt und die Reaktorrührung auf 250 UpM eingestellt. 0,7 g des in Beispiel 1 hergestellten Trägerkatalysators wurden in 25 $cm^3$ eines entaromatisierten Benzinschnittes mit dem Siedebereich 100 °C bis 120 °C suspendiert und die Suspension in den Reaktor gegeben. Der Reaktor wurde auf die Polymerisationstemperatur von 70 °C aufgeheizt (7,5 °C/min) und 1 h bei dieser Polymerisationstemperatur durch Kühlung des Reaktormantels gehalten. Gestoppt wurde die Polymerisation durch schnelles Abgasen der überschüssigen Monomeren. Das Polymer wurde im Vakuum getrocknet. Polymerausbeute, Katalysatoraktivität und Produktdaten sind der Tabelle 1 zu entnehmen.

Vergleichsbeispiele 2 und 3

Es wurde verfahren wie in den Beispielen 3 und 4, als Katalysator wurde jedoch der Trägerkatalysator aus Vergleichsbeispiel 1 verwendet. Die Ergebnisse sind in Tabelle 1 aufgeführt. Die Molmasse $M_w$ liegt bei Verwendung des nicht erfindungsgemäßen Katalysatorsystems deutlich tiefer als im direkten Vergleich mit den Beispielen 3 und 4, bei denen das erfindungsgemäße Katalysatorsystem zum Einsatz kam.

Vergleichsbeispiele 4 und 5

Analog zu Vergleichsbeispiel 1 wurde unter Verwendung von rac-Dimethylsilandiylbis(2-methyl-4-naphthyl-1-indenyl)zirkoniumdichlorid als Metallocenkomponente ein Trägerkatalysator hergestellt.
Die Polymerisationen mit diesem Trägerkatalysator wurden analog zu den Vergleichsbeispielen 2 und 3 durchgeführt. Die Ergebnisse sind in Tabelle 1 aufgeführt.

Beispiel 5

Ein trockener 24 $dm^3$ -Reaktor wurde mit Propylen gespült und mit 0,5 bar Wasserstoff beaufschlagt. Anschließend wurde der Reaktor mit 12 $dm^3$ flüssigem Propylen und 22 $cm^3$ einer hexanischen Triisobutylaluminiumlösung (8 mmol Al, 2 $cm^3$ Triisobutylaluminium verdünnt mit 20 $cm^3$ Hexan) befüllt und die Reaktorrührung auf 250 UpM eingestellt. 0,7 g des in Beispiel 1 hergestellten Trägerkatalysators wurden in 25 $cm^3$ eines entaromatisierten Benzinschnittes mit dem Siedebereich 100 °C bis 120 °C suspendiert und die Suspension in den Reaktor gegeben. Der Reaktor wurde auf die Polymerisationstemperatur von 70 °C aufgeheizt (7,5 °C/min) und 1 h bei dieser Polymerisationstemperatur durch Kühlung des Reaktormantels gehalten.
Anschließend wurde der Reaktor auf 10 bar entspannt und mit 20 bar Ethylen beaufschlagt. Der Ansatz wurde

bei einer Temperatur von 60 °C 2 h weiterpolymerisiert und dann durch rasches Abgasen der überschüssigen Monomeren gestoppt.

Es wurde ein Blockcopolymer mit den folgenden Eigenschaften erhalten:

Homopolymermatrix (iPP aus Fraktionierung)

Smp. = 157 °C, $M_w$ = 280 000 g/mol, $M_w/M_n$ = 2,6, VZ = 230 $cm^3/g$, Kautschuk (Ethylen-Propylen-Copolymer) $T_g$ = - 49 °C, 44 Gew% $C_2$, VZ = 374 $cm^3/g$, $M_w$ = 402 500 g/mol, $M_w/M_n$ = 3,0,

Vergleichsbeispiel 6

Das Beispiel 5 wurde unter Verwendung eines gemäß Vergleichsbeispiel 1 dargestellten Trägerkatalysators wiederholt.

Es wurde ein Blockcopolymer mit den folgenden Eigenschaften erhalten:

Homopolymermatrix (iPP aus Fraktionierung)

Smp. = 152 °C, $M_w$ = 167 500 g/mol, $M_w/M_n$ = 2,7, VZ = 147 $cm^3/g$, Kautschuk (Ethylen-Propylen-Copolymer) $T_g$ = - 48 °C, 42 Gew% $C_2$, VZ = 182 $cm^3/g$, $M_w$ = 224 000 g/mol, $M_w/M_n$ = 2,9,

Vergleichsbeispiel 7

Das Beispiel 5 wurde unter Verwendung eines gemäß Vergleichsbeispiel 4 dargestellten Trägerkatalysators wiederholt.

Es wurde ein Blockcopolymer mit den folgenden Eigenschaften erhalten:

Homopolymermatrix (iPP aus Fraktionierung)

Smp. = 154 °C, $M_w$ = 198 500 g/mol, $M_w/M_n$ = 2,6, VZ = 168 $cm^3/g$, Kautschuk (Ethylen-Propylen-Copolymer) $T_g$ = - 50 °C, 46 Gew% $C_2$, VZ = 280 $cm^3/g$, $M_w$ = 354 000 g/mol, $M_w/M_n$ = 2,7,

| Bsp. | Ausbeute [kg PP] | Aktivität [kg PP/g/h] | Smp. [°C] | VZ [$cm^3/g$] | $M_w$ [g/mol] | $M_w/M_n$ | $C_2$—Gehalt [Gew%] |
|---|---|---|---|---|---|---|---|
| 3 | 2,24 | 3,2 | 140 | 628 | 815 000 | 2,2 | 3,0 |
| 4 | 2,38 | 3,4 | 109 | 436 | 586 000 | 2,4 | 10,6 |
| V2 | 1,47 | 2,1 | 135 | 248 | 295 500 | 2,3 | 3,1 |
| V3 | 1,61 | 2,3 | 105 | 198 | 214 000 | 2,3 | 10,5 |
| V4 | 1,19 | 1,7 | 133 | 489 | 508 500 | 2,3 | 3,0 |
| V5 | 1,26 | 1,8 | 106 | 401 | 464 000 | 2,8 | 10,5 |

**Patentansprüche**

1. Katalysatorsysteme enthaltend mindestens ein Metallocen der Formel I

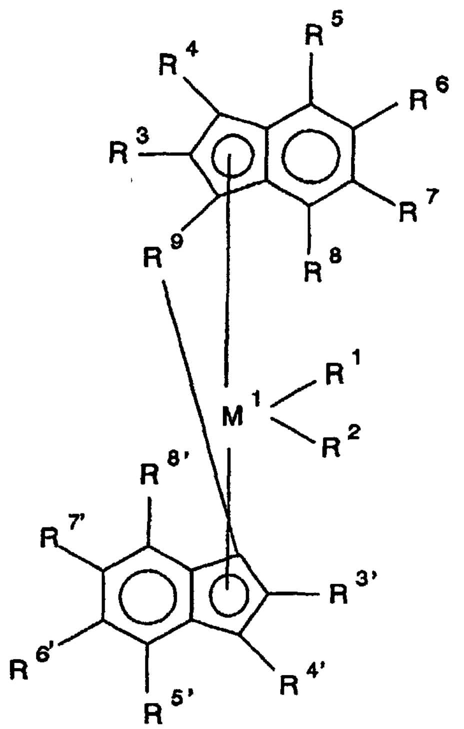

(I)

worin

| | |
|---|---|
| $M^1$ | ein Metall der Gruppe IVb des Periodensystems der Elemente ist, |
| $R^1$ und $R^2$ | gleich oder verschieden sind und ein Wasserstoffatom, eine $C_1$ bis $C_{10}$-Alkylgruppe, eine $C_1$ bis $C_{10}$-Alkoxygruppe, eine $C_6$ bis $C_{20}$-Arylgruppe, eine $C_6$ bis $C_{10}$-Aryloxygruppe, eine $C_2$ bis $C_{10}$-Alkenylgruppe, eine OH-Gruppe, eine $NR^{12}_2$-Gruppe, wobei $R^{12}$ eine $C_1$ bis $C_{10}$-Alkylgruppe oder $C_6$ bis $C_{14}$-Arylgruppe ist, oder ein Halogenatom bedeuten, |
| $R^3$,$R^4$,$R^6$,$R^7$, $R^8$ sowie $R^{3'}$, $R^{4'}$ , $R^{6'}$, $R^{7'}$ und $R^{8'}$ | gleich oder verschieden sind und ein Wasserstoffatom, eine Kohlenwasserstoffgruppe, die halogeniert, linear, cyclisch oder verzweigt sein kann, wie eine $C_1$ bis $C_{10}$-Alkylgruppe, $C_2$ bis $C_{10}$-Alkenylgruppe, $C_6$ bis $C_{20}$-Arylgruppe, eine $C_7$ bis $C_{40}$-Arylalkylgruppe, eine $C_7$ bis $C_{40}$-Alkylarylgruppe oder eine $C_8$ bis $C_{40}$-Arylalkenylgruppe bedeuten mit der Maßgabe, daß $R^3$ und $R^{3'}$ von Wasserstoff verschieden sind und |
| $R^5$ und $R^{5'}$ | gleich oder verschieden sind und eine $C_6$ bis $C_{40}$-Arylgruppe der Formel<br>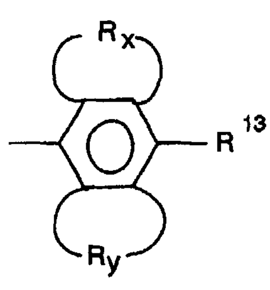 |

mit x, y = 0, 1 und x + y = 0, 1 oder 2 bedeuten, wobei das aromatische Ringsystem x und/oder das aromatische Ringsystem y auch mit den Resten $R^6$, $R^{6'}$ oder $R^4$, $R^{4'}$ verknüpft sein kann und die $C_6$ bis $C_{40}$-Arylgruppe in para-Position zur Bindungsstelle an den Indenylring einen Substituenten $R^{13}$ trägt und $R^{13}$ tert.-Butyl, Adamantyl oder $(F_3C)_3C$ ist,

$R^9$ eine Verbrückung

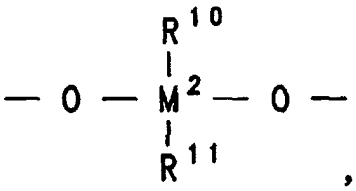,
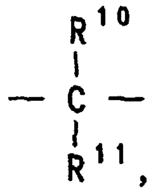,
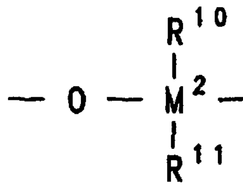,
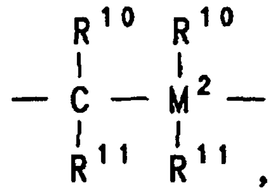,

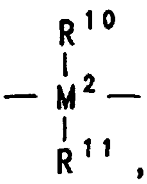,
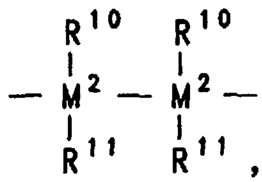,
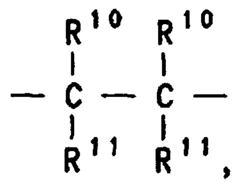,
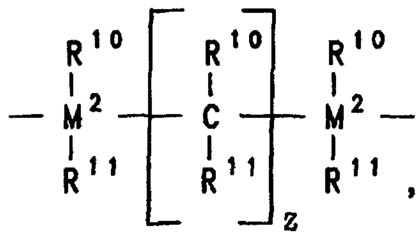,

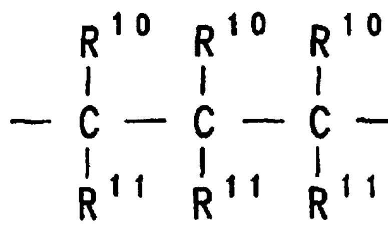,

>$BR^{10}$, >$AlR^{10}$, -Ga-, -O-, -S-, >SO, >$SO_2$, >$NR^{10}$, >OO, >$PR^{10}$ oder >$R(O)R^{10}$,
bedeutet, wobei

$R^{10}$ und $R^{11}$ auch bei gleicher Indizierung, gleich oder verschieden sein können und ein Wasserstoffatom, ein Halogenatom oder eine $C_1$ bis $C_{40}$-kohlenstoffhaltige Gruppe bedeuten, wie eine $C_1$ bis $C_{20}$-Alkyl , eine $C_1$ bis $C_{10}$-Fluoralkyl-, eine $C_1$ bis $C_{10}$-Alkoxy-, eine $C_6$ bis $C_{14}$-Aryl-, eine $C_6$ bis $C_{10}$-Fluoraryl-, eine $C_6$ bis $C_{10}$-Aryloxy-, eine $C_2$ bis $C_{10}$-Alkenyl-, eine $C_7$ bis $C_{40}$-Arylalkyl-, eine $C_7$ bis $C_{40}$-Alkylaryl- oder eine $C_8$ bis $C_{40}$-Arylalkenylgruppe oder $R^{10}$ und $R^{11}$ bilden jeweils mit den sie verbindenden Atomen einen oder mehrere Ringe, z eine ganze Zahl von Null bis 18 ist und

$M^2$ Silizium, Germanium oder Zinn bedeutet und

$R^9$ auch zwei Einheiten der Formel I miteinander verknüpfen kann,

sowie mindestens einen Cokatalysator und mindestens einen Träger.

**2.** Katalysatorsystem nach Anspruch 1, enthaltend mindestens eine Verbindung der Formel I, worin

| | |
|---|---|
| $M^1$ | Zirkonium, Hafnium oder Titan ist, |
| $R^1$ und $R^2$ | gleich sind und für Methyl oder Chlor stehen, |
| $R^3$ und $R^{3'}$ | gleich oder verschieden sind und eine Kohlenwasserstoffgruppe, die halogeniert, linear, cyclisch oder verzweigt sein kann, wie eine $C_1$ bis $C_{10}$-Alkylgruppe, $C_2$-$C_{10}$-Alkenylgruppe, eine $C_7$-$C_{40}$-Alkylarylgruppe bedeuten, |
| R9 | $R^{10}R^{11}Si=$, $R^{10}R^{11}Ge=$, $R^{10}R^{11}C=$ oder $-(R^{10}R^{11}C-CR^{10}R^{11})-$ bedeutet, wobei $R^{10}$ und $R^{11}$ gleich oder verschieden sind und eine $C_1$-$C_{20}$-Kohlenwasserstoffgruppe, insbesondere $C_1$-$C_{10}$-Alkylgruppe oder $C_6$-$C_{14}$-Arylgruppe bedeuten, |
| $R^5$ und $R^{5'}$ | gleich oder verschieden sind und eine $C_6$ bis $C_{20}$-Arylgruppe bedeuten, die in para-Position zur Bindungsstelle an den Indenylring einen Substituenten $R^{13}$ trägt. |

**3.** Katalysatorsystem nach Anspruch 1 oder 2, enthaltend mindestens eine Verbindung der Formel I, worin

| | |
|---|---|
| $M^1$ | Zirkonium ist, |
| $R^1$ und $R^2$ | gleich sind und für Methyl oder Chlor, insbesondere Chlor, stehen, |
| $R^9$ | $R^{10}R^{11}Si=$, $R^{10}R^{11}C=$ oder $-(R^{10}R^{11}C-CR^{10}R^{11})-$ ist, worin $R^{10}$ und $R^{11}$ gleich oder verschieden sind und Wasserstoff, Phenyl, Methyl oder Ethyl bedeuten, |
| $R^4$,$R^6$,$R^7$ und $R^8$ sowie $R^{4'}$, $R^{6'}$,$R^{7'}$und $R^{8'}$ | Wasserstoff sind, und |
| $R^5$ und $R^{5'}$ | gleich oder verschieden sind und eine in para-Position substituierte Phenyl-, Naphthyl- oder Anthracenylgruppe bedeuten. |

**4.** Katalysatorsystem nach einem oder mehreren der Ansprüche 1 bis 3, enthaltend mindestens eine Verbindung der Formel I, worin

| | |
|---|---|
| $M^1R^1R^2$ | $ZrCl_2$, $Zr(CH_3)_2$, |
| $R^3$,$R^{3'}$ | Methyl, Ethyl, Isopropyl, Isobutyl, n-Butyl, s-Butyl, |
| $R^4$,$R^8$,$R^{4'}$,$R^{8'}$ | Wasserstoff, |
| $R^6$,$R^7$,$R^{6'}$,$R^{7'}$ | Wasserstoff, $C_1$ bis $C_4$-Alkyl, $C_6$ bis $C_{10}$-Aryl, |
| $R^5$ und $R^{5'}$ | p-tert.-Butyl-phenyl, p-Adamantyl-phenyl, p-$(F_3C)_3C$-phenyl und |
| $R^9$ | Dimethylsilandiyl, Dimethylgermandiyl, Ethyliden, 1-Methylethyliden, 1,1-Dimethylethyliden, 1,2-Dimethylethyliden, 1,1,2,2-Tetramethylethyliden, Dimethylmethyliden bedeuten. |

**5.** Verfahren zur Herstellung eines frei fließenden Katalysatorsystems nach einem oder mehreren der Ansprüche 1 bis 4, wobei

a) eine Metallocen/Cokatalysatormischung in einem geeigneten Löse- oder Suspensionsmittel hergestellt wird,

b) die Metallocen/Cokatalysatormischung auf einen porösen, bevorzugt anorganischen dehydratisierten Träger aufgebracht wird,

c) das Lösungsmittel von der resultierenden Mischung entfernt wird,

d) das geträgerte Katalysatorsystem isoliert wird.

**6.** Verfahren zur Herstellung eines frei fließenden geträgerten Katalysatorsystems nach Anspruch 5, wobei

e) das enthaltene geträgerte Katalysatorsystem mit einem oder mehreren olefinischen Monomer(en) vorpolymerisiert wird.

**7.** Frei fließendes geträgertes Katalysatorsystem, erhältlich nach Anspruch 5 oder 6.

**8.** Verwendung eines Katalysatorsystems nach einem oder mehreren der Ansprüche 1 bis 7 zur Herstellung von

Polymeren mit hohem Schmelzpunkt.

**9.** Verfahren zur Herstellung eines Homopolymers und/oder Copolymers mit der bevorzugten Formel $R_m$-CH=CH-$R_n$ für das Monomer, worin $R_m$ und $R_n$ gleich oder verschieden sind und ein Wasserstoffatom oder eine kohlenstoffhaltige Gruppe mit 1 bis 20 C-Atomen, insbesondere 1 bis 10 C-Atome bedeuten und $R_m$ und $R_n$ zusammen mit den sie verbindenden Atomen einen oder mehrere Ringe bilden können, wobei mit einem Katalysatorsystem nach einem oder mehreren der Ansprüche 1 bis 7 polymerisiert wird.

**10.** Verfahren zur Herstellung eines Homo- und/oder Copolymers nach Anspruch 9, das in Lösung, in Masse, in Suspension oder in der Gasphase kontinuierlich oder diskontinuierlich, einoder mehrstufig durchgeführt wird.

**Claims**

**1.** A catalyst system comprising at least one metallocene of the formula I

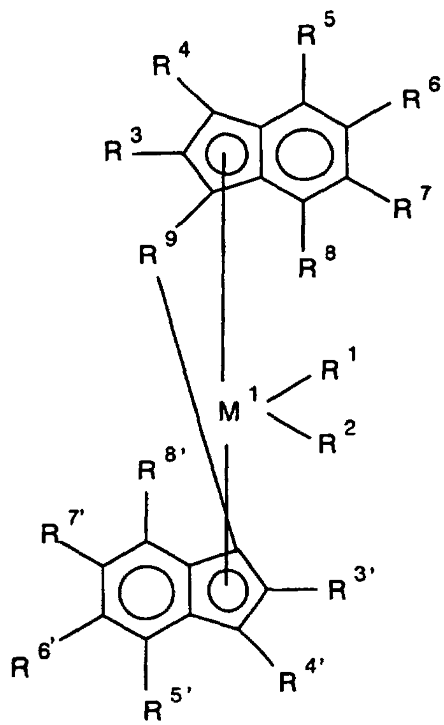

(I)

where

| | |
|---|---|
| $M^1$ | is a metal of Group IVb of the Periodic Table of the Elements, |
| $R^1$ and $R^2$ | are identical or different and are each a hydrogen atom, a $C_1$-$C_{10}$-alkyl group, a $C_1$-$C_{10}$-alkoxy group, a $C_6$-$C_{20}$-aryl group, a $C_6$-$C_{10}$-aryloxy group, a $C_2$-$C_{10}$-alkenyl group, an OH group, an $NR^{12}_2$ group, where $R^{12}$ is a $C_1$-$C_{10}$-alkyl group or $C_6$-$C_{14}$-aryl group, or a halogen atom, |
| $R^3$, $R^4$, $R^6$, $R^7$, $R^8$ and also $R^{3'}$, $R^{4'}$, $R^{6'}$, $R^{7'}$ and $R^{8'}$ | are identical or different and are each a hydrogen atom, a hydrocarbon group which may be halogenated, linear, cyclic or branched, for example a $C_1$-$C_{10}$-alkyl group, $C_2$-$C_{10}$-alkenyl group, $C_6$-$C_{20}$-aryl group, a $C_7$-$C_{40}$-aryla- |

lkyl group, a $C_7$-$C_{40}$-alkylaryl group or a $C_8$-$C_{40}$-arylalkenyl group, with the proviso that $R^3$ and $R^{3'}$ are not hydrogen, and

$R^5$ and $R^{5'}$ are identical or different and are each a $C_6$-$C_{40}$-aryl group of the formula

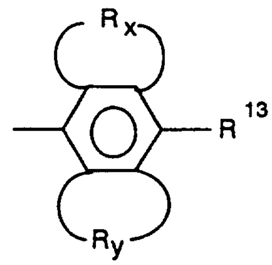

where x, y = 0, 1 and x + y = 0, 1 or 2, where the aromatic ring system x and/or the aromatic ring system y can also be linked to the radicals $R^6$, $R^{6'}$ or $R^4$, $R^{4'}$, and the $C_6$-$C_{40}$-aryl group bears a substituent $R^{13}$ in the para position to the bonding position to the indenyl ring and $R^{13}$ is tert-butyl, adamantyl or $(F_3C)_3C$,

$R^9$ is a bridge

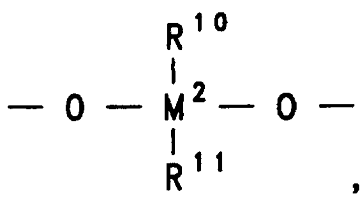 , 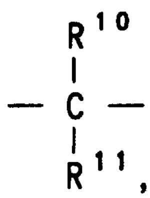 , 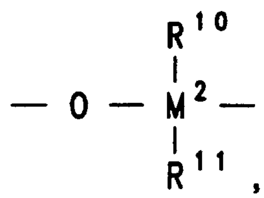 , 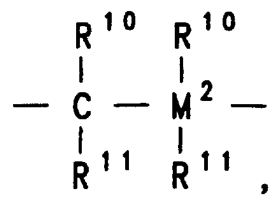 ,

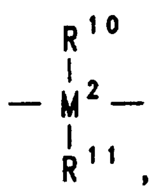 , 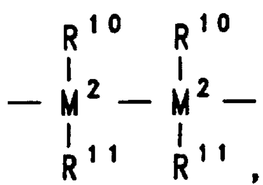 , 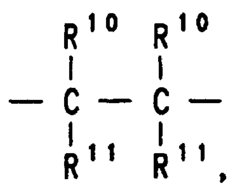 , 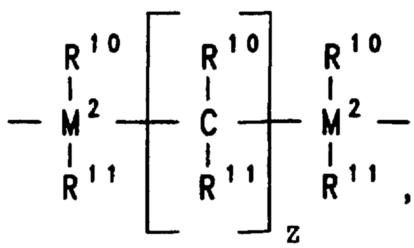 ,

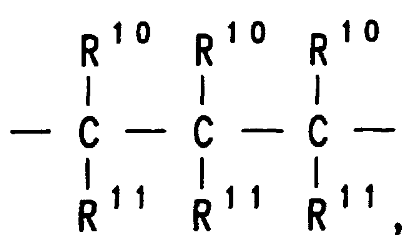 ,

$>$BR$^{10}$, $>$AlR$^{10}$, -Ga-, -O-, -S-, $>$SO, $>$SO$_2$, $>$NR$^{10}$, $>$OO, $>$PR$^{10}$ or $>$R(O)R$^{10}$,

where

| | |
|---|---|
| $R^{10}$ and $R^{11}$, | even when bearing the same index, can be identical or different and are each a hydrogen atom, a halogen atom or a $C_1$-$C_{40}$-group such as a $C_1$-$C_{20}$-alkyl group, a $C_1$-$C_{10}$-fluoroalkyl group, a $C_1$-$C_{10}$-alkoxy group, a $C_6$-$C_{14}$-aryl group, a $C_6$-$C_{10}$-fluoroaryl group, a $C_6$-$C_{10}$-aryloxy group, a $C_2$-$C_{10}$-alkenyl group, a $C_7$-$C_{40}$-arylalkyl group, a $C_7$-$C_{40}$-alkylaryl group or a $C_8$-$C_{40}$-arylalkenyl group or $R^{10}$ and $R^{11}$ together with the atoms connecting them form one or more rings, z is an integer from zero to 18 and |
| $M^2$ | is silicon, germanium or tin, and |
| $R^9$ | may also link two units of the formula I to one another, |

and at least one cocatalyst and at least one support.

**2.** A catalyst system as claimed in claim 1 comprising at least one compound of the formula I, wherein

| | |
|---|---|
| $M^1$ | is zirconium, hafnium or titanium, |
| $R^1$ and $R^2$ | are identical and are methyl or chlorine, |
| $R^3$ and $R^{3'}$ | are identical or different and are each a hydrocarbon group which may be halogenated, linear, cyclic or branched, for example a $C_1$-$C_{10}$-alkyl group, $C_2$-$C_{10}$-alkenyl group or a $C_7$-$C_{40}$-alkylaryl group, |
| $R^9$ | is $R^{10}R^{11}Si=$, $R^{10}R^{11}Ge=$, $R^{10}R^{11}C=$ or $-(R^{10}R^{11}C-CR^{10}R^{11})-$, where $R^{10}$ and $R^{11}$ are identical or different and are each a $C_1$-$C_{20}$-hydrocarbon group, in particular $C_1$-$C_{10}$-alkyl or $C_6$-$C_{14}$-aryl, |
| $R^5$ and $R^{5'}$ | are preferably identical or different and are each a $C_6$-$C_{20}$-aryl group which in the para position to the bonding position to the indenyl ring bears a substituent $R^{13}$. |

**3.** A catalyst system as claimed in claim 1 or 2 comprising at least one compound of the formula I, wherein

| | |
|---|---|
| $M^1$ | is zirconium, |
| $R^1$ and $R^2$ | are identical and are methyl or chlorine, in particular chlorine, |
| $R^9$ | is $R^{10}R^{11}Si=$, $R^{10}R^{11}C=$ or $-(R^{10}R^{11}C-CR^{10}R^{11})-$, where $R^{10}$ and $R^{11}$ are identical or different and are hydrogen, phenyl, methyl or ethyl, |
| $R^4$, $R^6$, $R^7$ and $R^8$ and also $R^{4'}$, $R^{6'}$, $R^{7'}$ and $R^{8'}$ | are hydrogen, and |
| $R^5$ and $R^{5'}$ | are identical or different and are each a phenyl, naphthyl or anthracenyl group which is substituted in the para position. |

**4.** A catalyst system as claimed in any of claims 1 to 3 comprising at least one compound of the formula I, wherein

| | |
|---|---|
| $M^1R^1R^2$ | is $ZrCl_2$, $Zr(CH_3)_2$, |
| $R^3$,$R^{3'}$ | are methyl, ethyl, isopropyl, isobutyl, n-butyl, s-butyl, |
| $R^4$,$R^8$,$R^{4'}$,$R^{8'}$ | are hydrogen, |
| $R^6$,$R^7$,$R^{6'}$,$R^{7'}$ | are hydrogen, $C_1$-$C_4$-alkyl, $C_6$-$C_{10}$-aryl, |
| $R^5$ and $R^{5'}$ | are p-tert-butylphenyl, p-adamantylphenyl, p-$(F_3C)_3C$-phenyl, and |
| $R^9$ | is dimethylsilanediyl, dimethylgermanediyl, ethylidene, 1-methylethylidene, 1,1-dimethylethylidene, 1,2-dimethylethylidene, 1,1,2,2-tetramethylethylidene, dimethylmethylidene. |

**5.** A process for preparing a free-flowing catalyst system as claimed in any of claims 1 to 4, wherein

a) a metallocene/cocatalyst mixture in a suitable solvent or suspension medium is prepared,

b) the metallocene/cocatalyst mixture is applied to a porous, preferably inorganic, dehydrated support,

c) the solvent is removed from the resulting mixture and

d) the supported catalyst system is isolated.

**6.** A process for preparing a free-flowing supported catalyst system as claimed in claim 5, wherein

e) the supported catalyst system obtained is prepolymerized with one or more olefinic monomer(s).

**7.** A free-flowing supported catalyst system obtainable as claimed in claim 5 or 6.

**8.** The use of a catalyst system as claimed in any of claims 1 to 7 for preparing polymers having a high melting point.

**9.** A process for producing a homopolymer or copolymer having the preferred formula $R_m$-CH=CH-$R_n$ for the monomer, where $R_m$ and $R_n$ are identical or different and are each a hydrogen atom or a group having from 1 to 20 carbon atoms, in particular from 1 to 10 carbon atoms, and $R_m$ and $R_n$ together with the atoms connecting them can form one or more rings, polymerization being performed using a catalyst system as claimed in any of claims 1 to 7.

**10.** A process for preparing a homopolymer and/or copolymer as claimed in claim 9 which is carried out in solution, in bulk, in suspension or in the gas phase, continuously or batchwise, in one or more stages.

**Revendications**

**1.** Système catalyseur contenant au moins un métallocène de formule I

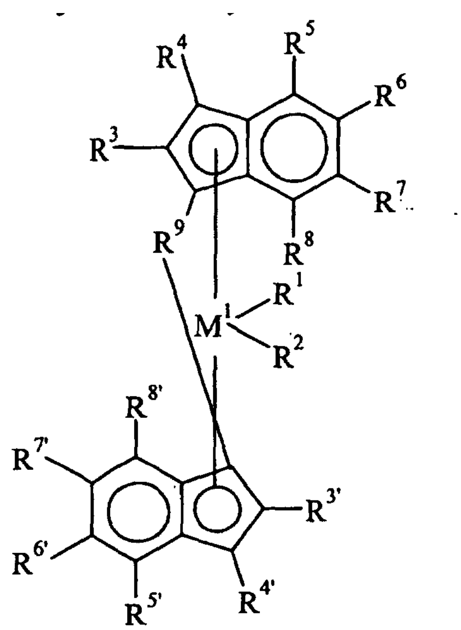

(I)

dans laquelle

$M^1$ est un métal du Groupe IVb du Tableau Périodique des Eléments,

$R^1$ et $R^2$ sont identiques ou différents et représentent chacun un atome d'hydrogène, un groupe alkyle en $C_1$ à $C_{10}$, un groupe alcoxy en $C_1$ à $C_{10}$, un groupe aryle en $C_6$ à $C_{20}$, un groupe aryloxy en $C_6$ à $C_{10}$, un groupe alcényle en $C_2$ à $C_{10}$, un groupe OH, un groupe $NR^{12}_2$, où $R^{12}$ est un groupe alkyle en $C_1$ à $C_{10}$ ou un groupe aryle en $C_6$ à $C_{14}$, ou encore un atome d'halogène,

$R^3$, $R^4$, $R^6$, $R^7$, $R^8$, ainsi que $R^{3'}$, $R^{4'}$, $R^{6'}$, $R^{7'}$ et $R^{8'}$ sont identiques ou différents et représentent chacun un atome d'hydrogène, un groupe hydrocarboné qui peut être halogéné, à chaîne droite ou ramifiée ou cyclique, tel qu'un groupe alkyle en $C_1$ à $C_{10}$, un groupe alcényle en $C_2$ à $C_{10}$, un groupe aryle en $C_6$ à $C_{20}$, un groupe arylalkyle en $C_7$ à $C_{40}$, un groupe alkylaryle en $C_7$ à $C_{40}$ ou un groupe arylalcényle en $C_8$ à $C_{40}$, à la condition que $R^3$ et $R^{3'}$ soient différents de l'hydrogène, et

$R^5$ et $R^{5'}$ sont identiques ou différents et représentent chacun un groupe aryle en $C_6$ à $C_{40}$

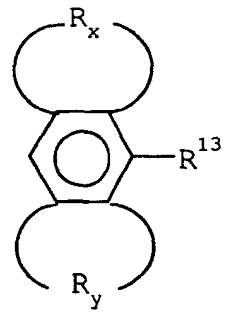

dans laquelle x, y = 0, 1 et x + y = 0, 1 ou 2, le système cyclique aromatique x et/ou le système cyclique aromatique y pouvant aussi être reliés aux radicaux $R^6$, $R^{6'}$ ou $R^4$, $R^{4'}$, et le groupe aryle en $C_6$ à $C_{40}$ porte en position para par rapport à la liaison au noyau indényle, un substituant $R^{13}$, et $R^{13}$ est un groupe tert-butyle, adamantyle ou $(F_3C)_3C$,

$R^9$ est un pont

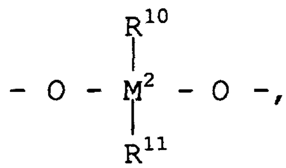 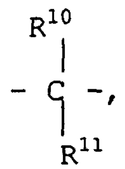 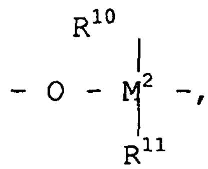 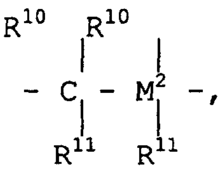

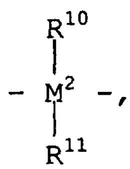 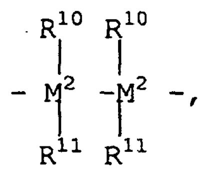 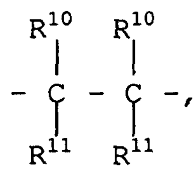

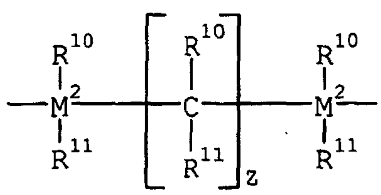

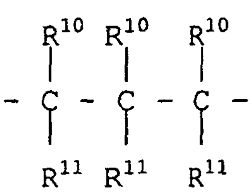

$>BR^{10}$, $>AlR^{10}$, -Ga-, -O-, -S-, >SO, $>SO_2$, $>NR^{10}$, >OO, $>PR^{10}$ ou $>R(O)R^{10}$,

où

$R^{10}$ et $R^{11}$, même avec les mêmes indices, peuvent être identiques ou différents et représentent chacun un atome d'hydrogène, un atome d'halogène ou un groupe hydrocarboné en $C_1$ à $C_{40}$, tel qu'un groupe alkyle en $C_1$ à $C_{20}$, un groupe fluoralkyle en $C_1$ à $C_{10}$, alcoxy en Ci à $C_{10}$, aryle en $C_6$ à $C_{14}$, fluoralkyle en $C_6$ à $C_{10}$, aryloxy en $C_6$ à $C_{10}$, alcényle en $C_2$ à $C_{10}$, arylalkyle en $C_7$ à $C_{40}$, alkylaryle en $C_7$ à $C_{40}$ ou arylalcényle en $C_8$ à $C_{40}$, ou encore $R^{10}$ et $R^{11}$, avec les atomes qui les relient, forment un ou plusieurs cycles, z est un nombre entier de 0 à 18, et

$M^2$ est le silicium, le germanium ou l'étain, et
$R^9$ peut aussi relier l'un à l'autre deux motifs de formule I
ainsi qu'au moins un co-catalyseur et au moins un support.

**2.** Système catalyseur selon la revendication 1, qui contient au moins un composé de formule I dans laquelle
$M^1$ est le zirconium, le hafnium ou le titane,
$R^1$ et $R^2$ sont identiques ou différents et représentent chacun le groupe méthyle ou chloro,
$R^3$ et $R^{3'}$ sont identiques ou différents et représentent chacun un groupe hydrocarboné qui peut être halogéné ou à chaîne droite ou ramifiée ou cyclique, tel qu'un groupe alkyle en $C_1$ à $C_{10}$, un groupe alcényle en $C_2$ à $C_{10}$, un groupe alkylaryle en $C_7$ à $C_{40}$,
$R^9$ est $R^{10}R^{11}Si=$, $R^{10}R^{11}Ge=$, $R^{10}R^{11}C=$ ou $-(R^{10}R^{11}C-CR^{10}R^{11})-$, où $R^{10}$ et $R^{11}$ sont identiques ou différents et représentent chacun un groupe hydrocarboné en $C_1$ à $C_{20}$, en particulier un groupe alkyle en $C_1$ à $C_{10}$ ou un groupe aryle en $C_6$ à $C_{14}$,
$R^5$ et $R^{5'}$ sont identiques ou différents et représentent chacun un groupe aryle en $C_6$ à $C_{20}$, qui portent en position para par rapport à la liaison au noyau indényle un substituant $R^{13}$.

**3.** Système catalyseur selon la revendication 1 ou 2, qui contient au moins un composé de formule I dans laquelle
$M^1$ est le zirconium,
$R^1$ et $R^2$ sont identiques ou différents et représentent chacun le groupe méthyle ou un atome de chlore, en particulier un atome de chlore,
$R^9$ est $R^{10}R^{11}Si=$, $R^{10}R^{11}C=$ ou $-(R^{10}R^{11}C-CR^{10}R^{11})-$, où $R^{10}$ et $R^{11}$ sont identiques ou différents et représentent chacun un atome d'hydrogène ou un groupe phényle, méthyle ou éthyle,
R4, $R^6$, $R^7$ et $R^8$, ainsi que $R^{4'}$, $R^{6'}$, $R^{7'}$ et $R^{8'}$, sont des atomes d'hydrogène, et
$R^5$ et $R^{5'}$ sont identiques ou différents et représentent chacun un groupe phényle, naphtyle ou anthracényle substitué en position para.

**4.** Système catalyseur selon l'une ou plusieurs des revendications 1 à 3, qui contient au moins un composé de formule I dans laquelle
$M^1R^1R^2$ est $ZrCl_2$, $Zr(CH_3)_2$,
$R^3$, $R^{3'}$ sont des groupes méthyle, éthyle, isopropyle, isobutyle, n-butyle, sec-butyle,
$R^4$, $R^8$, $R^{4'}$, $R^{8'}$ sont des atomes d'hydrogène,
$R^6$, $R^7$, $R^{6'}$, $R^{7'}$ sont des atomes d'hydrogène ou des groupes alkyle en $C_1$ à $C_4$ ou aryle en $C_6$ à $C_{10}$,
$R^5$ et $R^{5'}$ représentent chacun le groupe p-tert-butylphényle, p-adamantyl-phényle, p-$(F_3C)_3C$-phényle, et
$R^9$ est le groupe diméthylsilanediyle, diméthylgermanediyle, éthylidène, 1-méthyléthylidène, 1,1-diméthyléthylidène, 1,2-diméthyléthylidène, 1,1,2,2-tétraméthyléthylidène, diméthylméthylidène.

**5.** Procédé de préparation d'un système catalyseur à écoulement libre selon l'une ou plusieurs des revendications 1 à 4, dans lequel

a) on prépare un mélange métallocène/co-catalyseur dans un agent approprié de mise en solution ou en suspension,
b) on applique le mélange métallocène/co-catalyseur sur un support poreux, déshydraté, de préférence inorganique,
c) on élimine le solvant du mélange obtenu,
d) on isole le système catalyseur supporté.

**6.** Procédé de préparation d'un système catalyseur supporté à écoulement libre selon la revendication 5, dans lequel

e) on prépolymérise le système catalyseur supporté et présent avec un ou plusieurs monomères oléfiniques.

**7.** Système catalyseur supporté à écoulement libre pouvant être obtenu selon la revendication 5 ou 6.

**8.** Utilisation d'un système catalyseur selon l'une ou plusieurs des revendications 1 à 7 pour préparer des polymères à haut point de fusion.

**9.** Procédé de préparation d'un homopolymère et/ou d'un copolymère avec un monomère ayant la formule préférée $R_m-CH=CH-R_n$, où $R_m$ et $R_n$ sont identiques ou différents et représentent chacun un atome d'hydrogène ou un groupe hydrocarboné ayant de 1 à 20 et en particulier de 1 à 10 atomes de carbone, et $R_m$ et $R_n$ peuvent former

ensemble avec les atomes qui les relient un ou plusieurs cycles, auquel cas on polymérise avec un système catalyseur selon l'une des revendications 1 à 7.

10. Procédé de préparation d'un homo- et/ou d'un copolymère selon la revendication 9, procédé qui est mis en oeuvre en une ou plusieurs étapes, en solution, en masse, en suspension ou en phase gazeuse, d'une manière continue ou discontinue.